# EUROPEAN PATENT APPLICATION

(11) **EP 0 706 778 A1**
(43) Date of publication of application: **17.04.1996**
(21) Application number: 95250242.5
(22) Date of filing: 02.10.1995
(51) Int. Cl.: A61B 17/00, A61F 2/00

(54) **Applicator for introducing flexible areal implants through an operative channel**

(30) Priority: 13.10.1994 DE 4437820
(71) Applicant: Ethicon, Inc., Somerville, New Jersey 08876-0151 (US)
(72) Inventor: Schilder, Lothar, D 22767 Hamburg (DE)
(74) Representative: UEXKÜLL & STOLBERG

(57) **Abstract**

An applicator for introducing flexible areal implants through an operative channel, in particular for applying a net (N) during an inguinal hernia operation, comprises a guide tube (1) and a sliding device (2). The guide tube (1) can be placed with its distal end over the operative channel. The sliding device (2) is introduced into the guide tube (1) via the proximal end of the guide tube (1). An unfolding device (5) attached to the distal end of a shaft device (4) engages with the net (N) with unfolding elements (34, 34', 35, 35', 36, 36', 37, 37') which can be unfolded after emerging at the distal end of the guide tube (1).

## Description

The invention relates to an applicator for introducing flexible areal implants through an operative channel, in particular for applying a net during an inguinal hernia operation.

It is frequently the case with surgical operations that a flexible areal implant has to be introduced in the folded-up or closed state through an operative channel which is accessible from outside, and unfolded behind the operative channel. This is for example the case during an inguinal hernia operation. Here, a net, for example made from polypropylene, serves to stabilise the tissue which forms during the healing process and thus grows into the net. During the operation, the net has to be placed between the tissue through which the operative channel passes and the parts of the intestines which are brought back to their original position during the operation. It is very difficult to spread out the net behind the operative channel using traditional means because in particular the edge region of the net unfolded into its areal shape is scarcely accessible through the operative channel.

It is therefore the object of the invention to provide an applicator for introducing flexible areal implants through an operative channel, which can be used in particular for applying a net during an inguinal hernia operation and allows the implant to be introduced and unfolded rapidly and safely.

This object is achieved by an applicator for introducing flexible areal implants through an operative channel, in particular for applying a net during an inguinal hernia operation, having the features of claim 1. Advantageous configurations emerge from the dependent claims.

The applicator according to the invention has a guide tube which can be placed with its distal end over the operative channel. A sliding device, which comprises a shaft device and an unfolding device attached to its distal end, can be inserted into the guide tube via the proximal end of the guide tube. Upon moving the sliding device forward in the distal direction, the flexible areal implant to be applied is pushed in front of the unfolding device. When the unfolding device emerges at the distal end of the guide tube together with the implant, unfolding elements arranged on the unfolding device ensure the opening or unfolding of the implant, so that it adopts the intended areal shape. The applicator according to the invention can therefore be used quickly and reliably.

In a preferred version, the unfolding elements have elongated springs, preferably helical springs, which are bent backwards within the guide tube upon pushing forward to the distal end in proximal direction. After emerging at the distal end of the guide tube, the springs, which run transversely to the longitudinal axis of the guide tube in the relaxed state, relax. They unfold the flexible implant guided before them so that it assumes its intended areal shape. When the sliding device is then pulled back in proximal direction, the springs bend back in the distal direction on reentering the guide tube, whereby they can press against the implant. This construction is simply designed and cheap and moreover allows automatic unfolding of the implant at its proposed position.

The unfolding device preferably has a carrier element running in the direction of the longitudinal axis of the guide tube, from whose distal end region several helical springs offset relative to one another in the azimuth direction extend at an approximate right angle relative to the carrier element. If other helical springs extend from a region of the carrier element offset in proximal direction, which lie in the azimuth direction between the helical springs extending from the distal end region of the carrier element, the implant is unfolded in two successive steps. Firstly, the helical springs attached in the distal end region of the carrier element act, and only then the helical springs emerging later from the guide tube, starting from the region of the carrier element offset in proximal direction. When the sliding device is moved back in proximal direction, the sequence is reversed. In this way a uniform unfolding and also a uniform pressing against the implant is achieved.

The bending force necessary to bend back the helical springs for pushing through the guide tube is preferably in a range from 0.6 N to 1.0 N (this value relating to a single helical spring and a cylindrical guide tube having an internal diameter of 15 mm). Helical springs which satisfy this criterion are thus flexible, whereby injuries during the unfolding of the implant are avoided. On the other hand, such springs are rigid enough to readily change into the relaxed state, i.e. to allow the implant to be unfolded safely.

The unfolding device can preferably be detached from the shaft device. The shaft device can for example be conceived as a reusable element which can be sterilised, whilst the unfolding device is a disposable article which is thrown away after the operation. Sterilisation is in fact difficult, in particular when using helical springs, since they have to be cleaned mechanically beforehand, so the concept of the unfolding device as a disposable article brings advantages.

The sliding device is preferably pre-tensioned by a spring element relative to the guide tube in proximal direction. On moving the sliding device forward, the surgeon must then exert a force, in return for which, however, the sliding device automatically reverts to its starting position after the implant has been unfolded, which makes the overall procedure easier.

In an advantageous configuration, a support on which the areal implant can be placed prior to insertion into the guide tube is provided in the outer area of the proximal region of the guide tube. The implant can be spread out on the support. Upon inserting the sliding device into the guide tube, the unfolding elements, which are initially in the unfolded state, engage with the spread-out implant, i.e. press it into the guide tube in such a way that a reliable unfolding is ensured at the other end of the guide tube.

Preferably provided in the outer area of the distal region of the guide tube is a base part with which the guide tube can be supported over the operative channel. The base part can be provided with a recess which preferably extends as far as the guide tube. The recess makes it possible for the surgeon to explore the implantation area to close up against the guide tube.

The applicator according to the invention is described in more detail below with reference to an embodiment. The drawings show:
- Figure 1: a perspective view of the guide tube of an applicator according to the invention,
- Figure 2: a perspective view of the sliding device of an applicator according to the invention,
- Figure 3: a perspective view which illustrates the arrangement of the guide tube from Figure 1 with a net placed thereon prior to the sliding device from Figure 2 being inserted,
- Figure 4: a perspective view of the arrangement after insertion of the sliding device into the guide tube and
- Figure 5: a simplified schematic longitudinal section which illustrates the position of the unfolded net after application during an inguinal hernia operation.

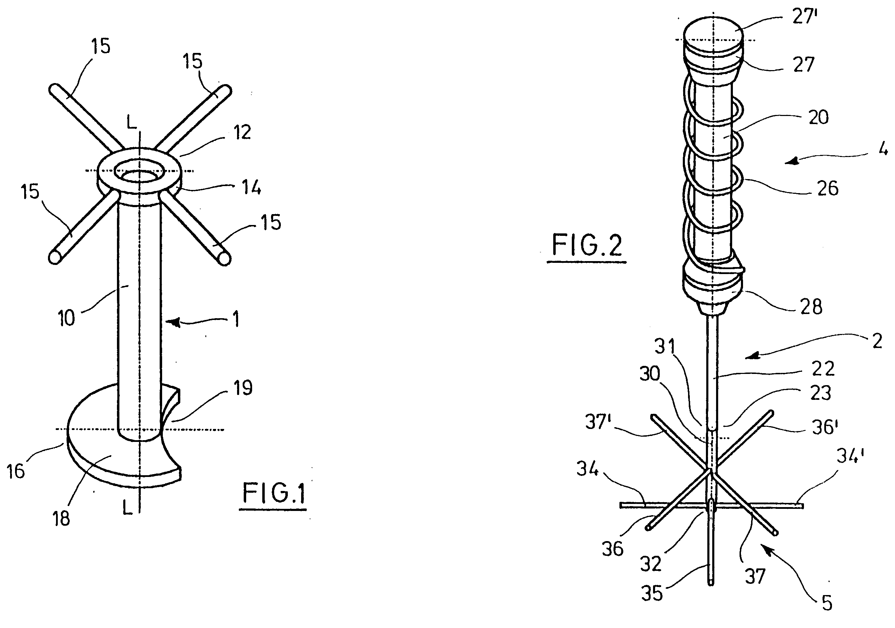

The applicator according to the invention has a guide tube 1 (see Figure 1) and a sliding device 2 with a shaft device 4 and an unfolding device 5 (see Figure 2).

The guide tube 1 consists essentially of a cylindrical tube piece 10. Attached to its proximal end 12 is a ring 14 from which four pins 15 extend. The pins 15 span a plane which stands perpendicular to the longitudinal axis L-L of the guide tube 1. The ring 14 and the pins 15 form a rest for placing the areal implant onto prior to insertion into the guide tube 1, see below. Attached to the distal end 16 of the guide tube 1 is a disk-shaped base part 18 which is provided with a recess 19 which extends as far as the tube piece 10. The inside of the guide tube 1 is freely passable.

The guide tube 1 is preferably made of high-grade steel, for example steel with the material number 1.4305. It can thus be adequately cleaned and sterilised.

The sliding device 2 shown in Fig. 2 has in its proximal region the shaft device 4 and in its distal region the unfolding device 5.

The shaft device 4 contains a cylindrical guide shaft 20, the outside diameter of which is matched to the internal diameter of the tube piece 10. A connecting part 22 running in the direction of the longitudinal axis of the sliding device 2, which has a smaller diameter than the guide shaft 20, extends from the distal end of the guide shaft 20. Provided at the distal end 23 of the connecting part 22 is a threaded pin (not recognizable from Figure 2) onto which the unfolding device 5 is screwed.

The guide shaft 20 is surrounded by a helical spring 26. The proximal end of the helical spring 26 is clamped in a circular groove which is provided on the outside of a collet 27. The inside of the collet 27 has an inner thread with which the collet 27 is screwed onto an outer thread arranged in the upper region of the guide shaft 20. A disk 27' firmly connected to the upper end of the guide shaft 20 serves as a limit stop for the collet 27. The distal end of the helical spring 26 is clamped in a circular groove which is provided on the outside of a slide ring 28 which can be moved along the guide shaft 20.

The guide shaft 20 preferably consists of high-grade steel (for example material number 1.4305), as does the helical spring 26 (for example material number 1.4310). High-grade steel which can be sterilised without problems is also suitable for the collet 27 and the slide ring 28.

The unfolding device 5 has a carrier element 30, running in the direction of the longitudinal axis of the sliding device 2, which is provided at its proximal face 31 with an inner thread so that it can be firmly screwed to the connecting part 22. Arranged in the proximity of the distal end 32 are two through bores, running at a right angle relative to the longitudinal axis of the carrier element 30, whose axes are perpendicular to one another. Fixed in the one bore, for example by means of a grub screw, is a helical spring 34, 34' and in the other bore a helical spring 35, 35'. The left-hand partial section 34 and the right-hand partial section 34' of the one helical spring each form an unfolding element. The same applies to the front partial section 35 and the rear partial section 35', which is concealed by the carrier element 30 in Figure 2, of the other helical spring.

A region of the carrier element 30 offset in proximal direction is provided with two further through bores, through which a helical spring with the partial pieces 36 and 36', and a helical spring with the partial pieces 37 and 37' run. These helical springs can be fixed for example likewise with the help of grub screws. The helical springs 36, 36' and 37, 37' run in the relaxed state at a right angle relative to the carrier element 30. They are however offset by 45° in the azimuth direction vis-à-vis the helical springs 34, 34' and 35, 35'.

The free ends of the partial sections 34, 34', 35, 35', 36, 36', 37, 37' of the helical springs are covered by end caps in order to rule out the risk of injury to the tissue coming into contact with these sections. These end caps can be manufactured from high-grade steel and pressed or welded onto the free ends of the springs. Other possibilities for covering the ends of the springs are however also conceivable, for example caps made from a thermoplastic could be welded on.

After an operation, the helical springs 34, 34', 35, 35', 36, 36' and 37, 37' can only be cleaned poorly. The entire unfolding device 5 is therefore preferably constructed as a disposable article. Considered as materials are for example plastic or high-grade steel (material number 1.4305 for the carrier element 30 and material number 1.4310 for the helical springs).

The helical springs 34, 34', 35, 35', 36, 36' and 37, 37' should be neither too rigid nor too flexible. Thus, on the one hand, injuries to the tissue are avoided when the helical springs suddenly change to the relaxed state (see below). On the other hand, it is to be ensured that the areal implant to be applied is unfolded safely. The bending force required to bend back the helical springs for sliding through the guide tube 1 should lie in the range from 0.6 N to 1.0 N (per partial section of spring). These values are relative to an internal diameter of the tube piece 10 measuring 15 mm, and can be converted accordingly to other diameters. A particularly favourable value is 0.8 N.

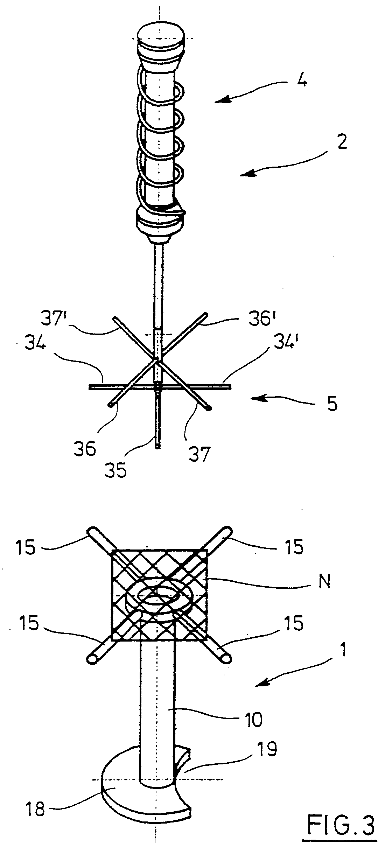

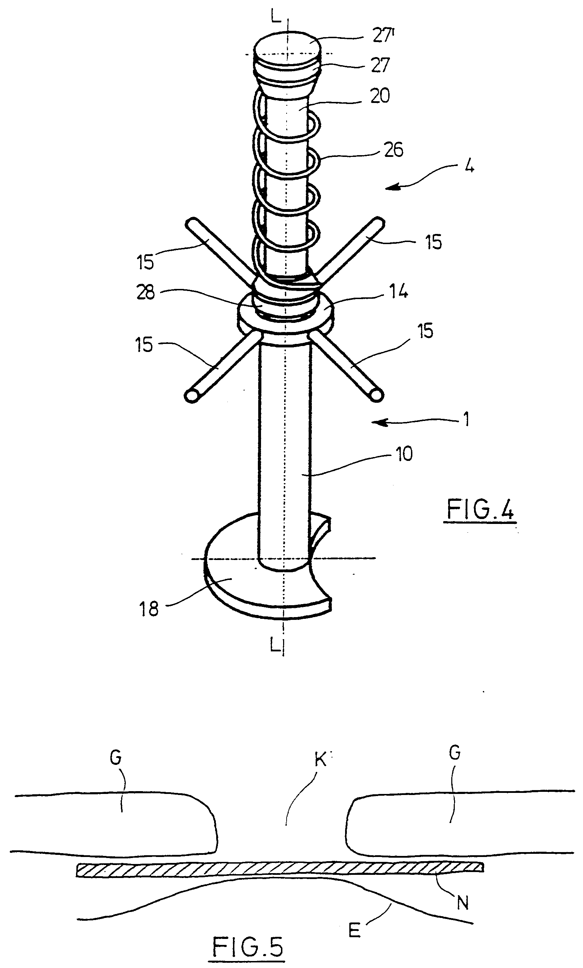

Shown in Figure 3 is the arrangement of the guide tube 1 and of the sliding device 2 when the applicator is starting to be used. It is the aim to apply a flexible areal implant, for example a net N made of polypropylene, through an operative channel K in such a way that net N is unfolded behind the operative channel K. Figure 5 shows in simplified longitudinal section the desired state after the operation when the unfolded net N lies between the tissue G surrounding the operative channel K and a section of intestines E.

On using the applicator, the guide tube 1 is firstly placed over the operative channel K. The recess 19 is of assistance when exploring the operative field. The net N is placed on the rest formed by the ring 14 and the pins 15, in the unfolded or spread-out state. The sliding device 2 is then moved against the guide tube 1, as shown in Figure 3. The helical springs 34, 34', 35, 35', 36, 36' and 37, 37', which form the unfolding elements, are in the relaxed state. When they touch the net N and then the sliding device 2 is moved further in the distal direction, they force the net N into the tube piece 10 and push it in front of them. Upon entering into the tube piece 10 the helical springs 34, 34', 35, 35', 36, 36' and 37, 37' bend back in proximal direction. As soon as the slide ring 28 is resting against the ring 14, the operator has to exert a force to move the sliding device 2 further forward, since now the helical spring 26 is pressing in the opposite direction. The pins 15 form an aid to firmly hold the guide tube 1. This situation is shown in Figure 4.

When the free ends of the helical springs 34, 34' and 35, 35' bent back in proximal direction have reached the distal end 16 of the guide tube 1, the distal end 32 of the carrier element 30 with the central region of the net N is already positioned at the distal end of the operative channel K or on the other side of it. On pushing them further forwards, the free ends of the helical springs 34, 34' and 35, 35' are no longer held by the tube piece 10, with the result that these helical springs relax, spreading the net N out. It is completely ensured that the net N is unfolded if the helical springs 36, 36' and 37, 37' also relax when the sliding device 2 moves further in the distal direction, i.e. if they change into the position shown in Figure 2.

If the surgeon now pulls the sliding device 2 back in proximal direction or allows it to slide back under the action of the helical spring 26, the movement of the helical springs 34, 34', 35, 35', 36, 36' and 37, 37' reverses. Firstly, the helical springs 36, 36' and 37, 37' enter into the tube piece 10, whereby they are bent over in the distal direction. They exert a force acting in the distal direction on the spread-out net N, as a result of which the net is pressed against securely. The helical springs 34, 34' and 35, 35' subsequently act in the same way in the regions of the net N which lie in between in the azimuth direction.

After the end of the operation, the unfolding device 5 can be unscrewed and disposed of. The guide tube 1 and the shaft device 4 are preferably cleaned and sterilised, so that they can be used again. In order to clean the shaft device 4, the collet 27 with the helical spring 26 and the slide ring 28 can be unscrewed and removed.

## Claims

1. Applicator for introducing flexible areal implants through an operative channel (K), in particular for applying a net (N) during an inguinal hernia operation, having
- a guide tube (1) with a proximal (12) and a distal (16) end, wherein the guide tube (1) can be placed with its distal end (16) above the operative channel (K), and
- a sliding device (2) which can be introduced via the proximal end (12) of the guide tube (1) into the guide tube (1), and which has at the distal end (23) of a shaft device (4) an unfolding device (5) with unfolding elements (34, 34', 35, 35', 36, 36', 37, 37') which can be unfolded after emerging at the distal end (16) of the guide tube (1).

2. Applicator according to claim 1, characterized in that the unfolding elements comprise elongated springs (34, 34', 35, 35', 36, 36', 37, 37') which are bent back in a proximal direction within the guide tube (1) upon being pushed forward onto the distal end (16) and which, after emerging at the distal end (16) of the guide tube (1), in the relaxed state run transversely to the longitudinal axis (L-L) of the guide tube (1).

3. Applicator according to claim 2, characterized in that the springs are helical springs (34, 34', 35, 35', 36, 36', 37, 37').

4. Applicator according to claim 3, characterized in that the free ends of the helical springs (34, 34', 35, 35', 36, 36', 37, 37') are covered by end caps.

5. Applicator according to claim 3 or 4, characterized in that the unfolding device (5) has a carrier element (30), running in the direction of the longitudinal axis (L-L) of the guide tube (1), from whose distal end region several helical springs (34, 34', 35, 35'), offset relative to one another in the azimuth direction, extend at approximately a right angle relative to the carrier element (30).

6. Applicator according to claim 5, characterized in that further helical springs (36, 36', 37, 37') extend from a region of the carrier element (30) offset in proximal direction, which lie in the azimuth direction between the helical springs (34, 34', 35, 35') extending from the distal end region of the carrier element.

7. Applicator according to one of claims 3 to 6, characterized in that the bending force required to bend back the helical springs (34, 34', 35, 35', 36, 36', 37, 37') for pushing through the guide tube (1), relative to an individual helical spring and a cylindrical guide tube having an internal diameter of 15 mm, lies in the range from 0.6 N to 1.0 N.

8. Applicator according to one of the preceding claims, characterized in that the unfolding device (5) can be removed from the shaft device (4).

9. Applicator according to one of the preceding claims, characterized in that the cross-sectional area of at least one section (20) of the shaft device (4) is matched to the area of the free cross-section of the guide tube (1).

10. Applicator according to one of the preceding claims, characterized in that the sliding device (2) is pretensioned by a spring element (26) relative to the guide tube (1) in proximal direction.

11. Applicator according to claim 10, characterized in that the spring element (26) is attached to the shaft device (4) in removable manner.

12. Applicator according to one of the preceding claims, characterized in that a support (14, 15) is provided in the outer area of the proximal region of the guide tube (1) for placing the areal implant (N) onto prior to insertion into the guide tube (1).

13. Applicator according to one of the preceding claims, characterized in that a base part (18) is provided in the outer area of the distal region of the guide tube (1) to support the guide tube (1) above the operative channel (K).

14. Applicator according to claim 13, characterized in that the base part (18) is provided with a recess (19) for exploring the implantation region.
